# EUROPEAN PATENT SPECIFICATION

(11) **EP 4 469 073 B1**
(45) Date of publication and mention of the grant of the patent: **04.11.2026**
(21) Application number: 23701435.2
(22) Date of filing: 19.01.2023
(51) Int. Cl.: A61K 38/05, A61K 38/55, A61K 45/06, A61P 31/14

(54) **COMPOUND FOR USE IN PREVENTING AND/OR TREATING AN INFECTION CAUSED BY SARS-COV-2**
VERBINDUNG ZUR VERWENDUNG BEI DER PRÄVENTION UND/ODER BEHANDLUNG EINER DURCH SARS-COV-2 VERURSACHTEN INFEKTION
COMPOSÉ À UTILISER POUR LA PRÉVENTION ET/OU LE TRAITEMENT D'UNE INFECTION CAUSÉE PAR LE SARS-COV-2

(30) Priority: 24.01.2022 EP 22305072
(43) Date of publication of application: 04.12.2024
(73) Proprietor: INSERM (Institut National de la Santé et de la Recherche Médicale), 75013 Paris (FR); Université Paris Cité, 75006 Paris (FR)
(72) Inventor: LAFORGE, Mireille, 75019 PARIS (FR); GRESSENS, Pierre, 75019 PARIS (FR)
(74) Representative: Ipsilon
(86) International application number: PCT/EP2023/051296
(87) International publication number: WO 2023/139182

(56) References cited:
- WO-A1-2009/092897
- WO-A1-2021/228846
- WO-A1-2022/008597
- WO-A1-2022/123062
- ZAKI ADHAM M M; AHMED YASMIN MOUSTAFA; ABDELHAFEZ ELSHIMAA M N: "CANDIDATURE OF THE SYNTHETIC CASPASE INHIBITORS AS NEW ANTI-SARS- COV-2 DRUG DISCOVERY, IN-SILICO MOLECULAR DOCKING", INTERNATIONAL JOURNAL OF PHARMACEUTICAL SCIENCES AND RESEARCH,, 1 January 2021 (2021-01-01), pages 104 - 119, XP055880018

## Description

### TECHNICAL FIELD

The present invention belongs to the field of the treatment and/or prevention of viral infection and more particularly of viral infection caused by SARS-CoV-2.

Indeed the present invention concerns a particular compound which is N-(2(quinolyl)-valyl-O-methylaspartyl-(2,6-difluorophenoxy)methyl ketone or a pharmaceutically acceptable salt or solvate thereof and a pharmaceutical composition and kit comprising such a compound for use in treating and/or preventing infection caused by SARS-CoV-2.

### STATE OF PRIOR ART

Coronaviruses are single-stranded, enveloped RNA viruses that belong to the Coronaviridae family.

Since the early 2000s, several severe infections caused by coronaviruses and affecting humans have appeared. Indeed, the severe acute respiratory syndrome coronavirus (SARS-CoV) was firstly identified in China in November 2002 while another coronavirus causing severe and sometimes fatal respiratory tract infections in humans was detected in June 2012 in Saudi Arabia and was named as the Middle East Respiratory Syndrome coronavirus (MERS-CoV).

In December 2019 an outbreak of pneumonia cases caused by a novel coronavirus occurred in Wuhan in China and spread quickly worldwide. The World Health Organization (WHO) named the corresponding coronavirus firstly as 2019-nCOV and then as SARS-CoV-2 and the related disease as Coronavirus disease 2019 (COVID-19). Since the disease spread quickly worldwide, the WHO declared it as a pandemic on 11 March 2020.

At the level of its genome sequence, the firstly identified SARS-CoV-2 presents around 80% nucleotide identity with SARS-CoV and 50% with MERS-CoV. In addition, SARS-CoV-2 was rapidly considered as less severe but more contagious than SARS-CoV and MERS-CoV.

Most people infected with SARS-CoV-2 present mild to moderate respiratory illness with at least one of the following symptoms: fever, headache, muscle pain, fatigue, dry cough, dyspnea, loss of taste and loss of smell but recover without requiring special treatments. Nevertheless, older people and those for which COVID-19 is associated to another medical disease or trouble such as, for example, obesity, cardiovascular disease, diabetes, chronic respiratory disease, and cancer may develop serious illness. In these cases, the illness may progress to pneumonia, also known as COVID-19 associated pneumonia or COVID-19 pneumonia and/or to multi-organ failure. Complications of COVID-19 may include acute respiratory distress syndrome (ARDS), acute respiratory failure and liver or cardiac injury.

In view of the above, numerous companies and public research organizations have sought to identify anti-viral compounds capable of preventing and/or treating infection caused by SARS-CoV-2.

In the International application WO 2021/228846, the present inventor has already proposed, as a potential anti-SARS-CoV-2 compound, the N-(2(quinolyl)-valyl-glutamyl(2,6-difluorophenoxy)methyl ketone. The latter is also known as "QVE-OPh", "Quinolyl-Val-Glu-OPh" or "QVD-OPh negative control". Indeed, the QVD-OPh namely N-(2(quinolyl)-valyl-aspartyl-(2,6-difluorophenoxy)methyl ketone is chemically very close to QVE-OPh because both compounds differ from each other by the side chain of one amino acid which is aspartic acid (Asp or D) for QVD-OPh and glutamic acid (Glu or E) for QVE-OPh. In terms of activity, QVD-OPh is a broad caspase inhibitor while QVE-OPh presents no caspase inhibitory activity and thus is used as a negative control thereof.

QVD-OPh can also be implemented in a methylated form and more particularly in an O-methylated form. In this form designated in the present disclosure as QVDM-OPh, the hydrogen atom of the carboxyl group in the side chain of the aspartic acid is substituted by a methyl group via an esterification reaction. QVD-OPh has already been proposed as an anti-viral compound in the International application WO 2009/092897. More particularly, the experimental data provided in this application have shown that QVD-OPh inhibits the apoptopic phenotype of the HIV-infected cells thanks to its caspase inhibitory activity and inhibits also the viral replication. The potential anti-HIV activity of QVD-OPh has been confirmed by Laforge et al, 2018 (J. Clin. Invest., vol. 128, pages 1627-1640) in which AIDS disease progression is prevented in SIV-infected rhesus macaques thanks to a QVD-OPh treatment which makes it possible a long-term control of viral replication. Nevertheless, no data on other types of viruses was given either in the International application WO 2009/092897, or in Laforge et al, 2018.

Even if QVE-OPh does not present any caspase inhibitory activity, the present inventor has demonstrated in the International application WO 2021/228846 that this compound is able to inhibit viral replication and more particularly HIV viral replication and SARS-CoV-2 viral replication. Indeed this compound is highly effective in the control of an *in vitro* infection caused by SARS-CoV-2 by inhibiting its replication inside the cell and by preventing viral production and new infections without any toxicity. The experimental data on anti-SRAS-CoV-2 activity of QVE-OPh are only *in vitro* data. In addition, it should be noted that this application compares QVE-OPh with QVD-OPh and QVDM-OPh only for the experiments concerning HIV.

The present inventor aims to identify additional anti-SARS-CoV-2 compound(s) with improved properties.

### SUMMARY OF THE INVENTION

The present invention enables the purpose set by the inventor to be reached.

In fact, the inventor has shown both *in vitro* and *in vivo* that QVDM-OPh presents an inhibitory activity against SARS-CoV-2. More particularly, this compound is effective in the control of SARS-CoV-2 infection *in vitro* by inhibiting the viral replication inside the cells and by preventing *in vitro* viral production and new infection without any toxicity. In addition, the anti-SRAS-CoV-2 activity of QVDM-OPh has been confirmed *in vivo* in hamsters suffering from COVID-19, for which the viral replication in their lungs is significantly reduced by a QVDM-OPh treatment. At the time of filing the present application, it was not at all obvious that QVDM-OPh would have such an activity. Even though QVE-OPh was described as an anti-HIV and anti-SARS-CoV-2 compound in the International application WO 2021/228846, this did not imply that QVDM-OPh known for its anti-HIV activity undoubtedly has an anti-SARS-CoV-2 activity. Indeed, on the one hand, HIV and SARS-CoV-2 belong to two very different types of viruses, namely retroviruses and coronaviruses, and on the other hand, QVD-OPh and QVDM-OPh are defined as antivirals with a limited spectrum (page 6, lines 8-10 of the International application WO 2021/228846).

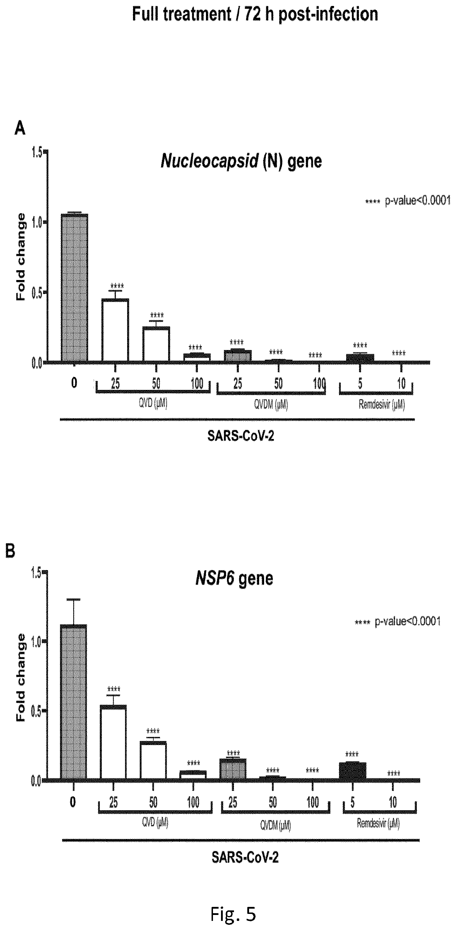

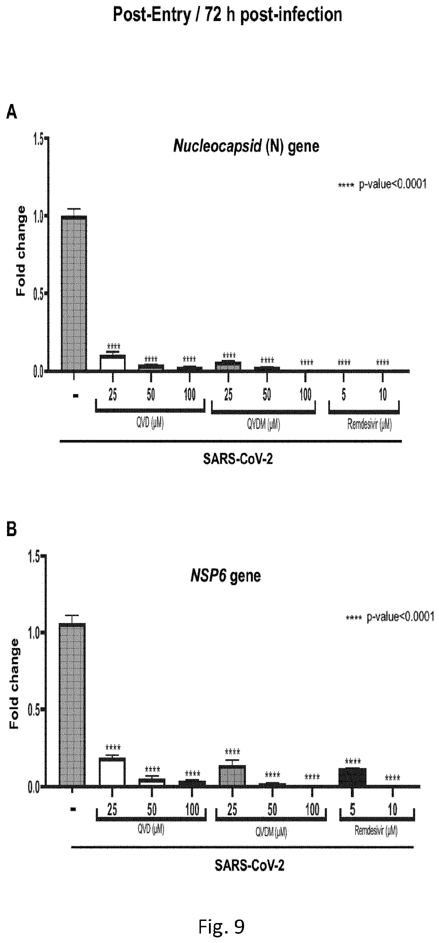

Even more surprisingly, there was no indication that QVDM-OPh would present a stronger anti-SARS-CoV-2 activity than QVD-OPh, as illustrated for the prevention of viral production and new infection in Example 1 below (Figures 5 and 9). Regarding the differences between QVD-OPh and QVDM-OPh, it is stated in the International application WO 2009/092897 that compounds without O-methylation are less hydrophobic which facilitates their use in aqueous media (paragraph [057]) and that QVD-OPh is less stable than QVDM-OPh (paragraph [0068]). However, no difference in their anti-viral activity was either mentioned, or suggested in the prior art. In particular, a same anti-viral effect is observed for QVE-OPh, QVD-OPh and QVDM-OPh on the viral replication of the HIV in the International application WO 2021/228846 (page 30, lines 24-32).

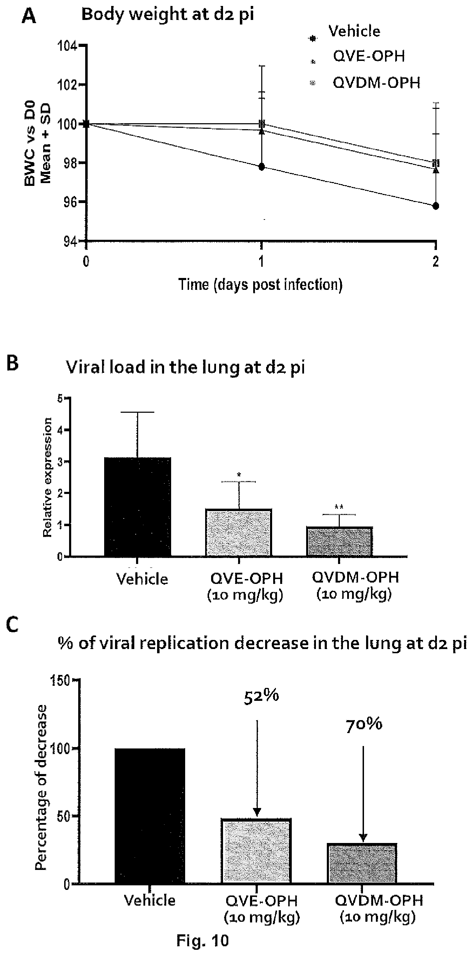

In addition, the present inventor has shown that QVDM-OPh presents an anti-SRAS-Cov-2 activity stronger that QVE-OPh *in vivo* in hamsters suffering from COVID-19 as confirmed by the compared level of viral load and viral replication in their lungs (Figure 10).

As a consequence, the present invention concerns a compound of formula (I): 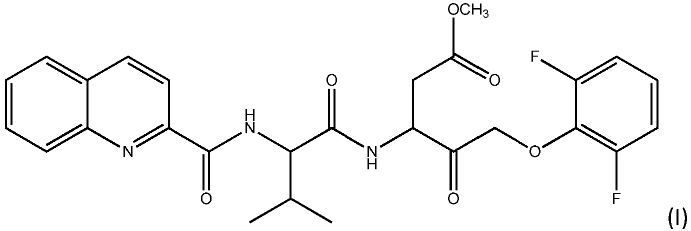 or a pharmaceutically acceptable salt or solvate thereof, for use in preventing and/or treating a viral infection caused by SARS-CoV-2.

The chemical name of the compound of formula (I) is N-(2(quinolyl)-valyl-O-methylaspartyl-(2,6-difluorophenoxy)methyl ketone i.e. QVDM-OPh, the O-methylated form of QVD-OPh. The compound of formula (I) is a pan-caspase inhibitor commercially available from Avantor^{™} delivered by VWR^{™} under the references BIOV2787-1 and BIOV2787-5.

The expression "pharmaceutically acceptable salt" of the compound of formula (I) means a salt that is pharmaceutically acceptable and that possesses essentially similar biological activity compared to the biological activity of the compound of formula (I) i.e. compared to the anti-SARS-CoV-2 activity of the compound of formula (I). It is clear that the pharmaceutically acceptable salt of the compound of formula (I) is to be non-toxic. Typically, the pharmaceutically acceptable salt implemented in the present invention is any acid addition salt obtained from the compound of formula (I). This acid addition salt may be a mineral acid addition salt or an organic acid addition salt. As illustrative and non-limiting examples of such acid addition salts, one can cite hydrochloride, chloride, hydrobromide, bromide, phosphate, hydrogen phosphate, dihydrogen phosphate, sulphate, bisulphate, borate, acetate, bitartrate, carbonate, citrate, formate, lactate, nitrate, oxalate, stearate and succinate salts. More particularly, the pharmaceutically acceptable salt of the compound of formula (I) implemented in the present invention is hydrochloride salt. This salt may be obtained by using hydrogen chloride, the latter being able to complex with at least one nitrogen atom of the compound of formula (I).

The expression "pharmaceutically acceptable solvate" of the compound of formula (I) means a molecular complex comprising the compound of formula (I) and stoichiometric or sub-stoichiometric amounts of one or more pharmaceutically acceptable solvent molecules such as, for example, ethanol or water. The term "hydrate" refers to when said solvent is water.

The pharmaceutically acceptable salt or solvate of the compound of formula (I) can be prepared by techniques well-known in the art, such as, for example, techniques involving precipitation step, filtration step, crystallization step, evaporation step, lyophilisation step and/or ion exchange resins.

The compound implemented in the invention or any pharmaceutically acceptable salt or solvate thereof is used for the prevention and/or the treatment of a viral infection caused by SARS-CoV-2.

The terms "treat", "treating" and "treatment", as used herein, are meant to include alleviating, attenuating or abrogating a condition or a disease, in particular, a viral infection caused by SARS-CoV-2 and/or the signs, symptoms and/or complications associated therewith. The signs or symptoms associated with a condition or a disease may be biochemical, cellular, histological, functional or physical, subjective or objective ones. The complications associated with a condition or a disease, in particular, a viral infection caused by SARS-CoV-2 may be COVID-19 pneumonia, acute respiratory distress syndrome (ARDS), acute respiratory failure and liver or cardiac injury.

The terms "prevent", "preventing" and "prevention" as used herein, are meant to include not only delaying or precluding the onset of a condition or disease, in particular, a viral infection caused by SARS-CoV-2 and/or the signs, symptoms and/or complications associated therewith but also barring a patient from acquiring a condition or disease, in particular, a viral infection caused by SARS-CoV-2, or reducing a patient's risk of acquiring a condition or disease, in particular, a viral infection caused by SARS-CoV-2.

The term "SARS-CoV-2", as used herein, is to be understood not only the firstly identified SARS-CoV-2 but also any variant or mutant thereof. The firstly identified SARS-CoV-2 is the initially discovered strain of the virus and the latter is also known as 2019-nCoV, HCoV-19, SARS2, COVID-19 virus, Wuhan coronavirus, Wuhan seafood market pneumonia virus and Human coronavirus 2019. The complete genome of this coronavirus (29903 bp ss-RNA) is accessible from the NCBI ("National Center for Biotechnology Information") site https://www.ncbi.nlm.nih.gov/ under the reference sequence NC_045512.2.

A "mutant or variant" of the firstly identified SARS-CoV-2 may be any mutant or variant already identified in at least some countries such as the variant Alpha (also known as the variant B.1.1.7), the variant Beta (also known as the variant B.1.351), the variant Gamma (also known as the variant P.1), the variant Delta (also known as the variant B.1.617.2), and the variant Omicron (also known as the variant B.1.1.529) and its sub-lineages (also known as the variants BA.1, BA.2, BA.3, BA.4 and BA.5). A "mutant or variant" of the firstly identified SARS-CoV-2 also encompasses any mutant or variant the complete genome of which presents at least 85%, at least 90%, at least 95%, at least 96%, at least 97%, at least 98% or even at least 99% identity with the reference sequence NC_045512.2.

The expression "identity percent between two nucleotide sequences" as used herein, is meant a percent of identical nucleotide residues between the compared two sequences, this percent being obtained after implementing the best alignment (optimum alignment) between both sequences. Those skilled in the art know different techniques enabling such an identity percent to be obtained and involving homology algorithms or computer programs such as the program BLAST.

The identity percent is statistic and the differences between both sequences are randomly distributed along these sequences. The differences between both sequences may consist of different modification types of the sequences: deletions, substitutions or additions of nucleotide residues.

The compound implemented in the invention or any pharmaceutically acceptable salt or solvate thereof implemented in the invention may be formulated as a pharmaceutical composition. Consequently, the present invention also concerns a pharmaceutical composition comprising, as active ingredient, the compound of formula (I) or a pharmaceutically acceptable salt or solvate thereof as previously defined for use in preventing and/or treating a viral infection caused by SARS-CoV-2.

Typically, the pharmaceutical composition implemented in the present invention further comprises at least one pharmaceutically acceptable vehicle.

The expression "pharmaceutically acceptable vehicle" as used herein is meant any substance which is added to the active ingredient implemented in the invention to promote its transport, avoid its substantial degradation in said composition and/or increase its half-life. Advantageously, such a pharmaceutically acceptable vehicle is sterile and non-pyrogenic and refers to molecular entities and compositions that do not produce an adverse, allergic or other untoward reaction when administered to an animal, in particular, a mammal, especially a human, as appropriate. It is chosen depending on the type of application of the pharmaceutical composition of the invention and in particular as a function of its administration mode. Advantageously, a pharmaceutically acceptable vehicle refers to a non-toxic, solid, semi-solid or liquid carrier, filler, diluent, additive, excipient, buffer, encapsulating material or formulation auxiliary of any type.

The pharmaceutical composition implemented in the invention can be administered by the systemic route; by the parenteral route, for example the intravenous, intra-arterial, intraperitoneal, intrathecal, intraventricular, intrasternal, intracranial, intramuscular or sub-cutaneous route; by the topical route; by the ocular route; by the oral route; and by the mucosal route such as the buccal, nasal, intranasal, rectal and vaginal routes.

As a solid pharmaceutical composition for oral administration implemented is the present invention, tablets, pills, powders, granules or capsules can be used where the active ingredient is mixed with one or more conventionally used inert diluents such as, for example, starch, calcium carbonate, sucrose, lactose, or gelatin, and possibly other substances such as, for example, a lubricant which may be magnesium stearate or talc, a colorant, or a coating.

As a liquid pharmaceutical composition for oral or ocular administration implemented is the invention, pharmaceutically acceptable, suspensions, solutions, emulsions, syrups containing conventionally used inert diluents, and possibly other substances such as, for example, wetting products, humectants, sweetening agents, flavoring agents, preservatives, or thickeners can be used.

The sterile pharmaceutical composition for parenteral administration implemented in the invention can be sterilized, aqueous or non-aqueous solution, suspension or emulsion. As a solvent or vehicle, water, propylene-glycol, polyethylene glycol, plant oils, injectable ester like ethyl oleate or other suitable organic solvents can be used. This composition can also contain adjuvants, such as wetting agents, isotonising agents or emulsifiers.

The pharmaceutical composition for topic administration implemented in the invention can be, for example, creams, lotions, oral sprays, nose or eye drops or aerosol.

In a particular embodiment, the pharmaceutical composition implemented in the invention further comprises at least one additional therapeutic agent.

Thus, the pharmaceutical composition implemented in the invention comprises or consists of (i) the compound of formula (I) or a pharmaceutically acceptable salt or solvate thereof as previously defined, (ii) at least one pharmaceutically acceptable vehicle and optionally (iii) at least one additional therapeutic agent. In particular, the pharmaceutical composition implemented in the invention comprises or consists of (i) the compound of formula (I) or a pharmaceutically acceptable salt or solvate thereof as previously defined, (ii) at least one pharmaceutically acceptable vehicle and (iii) at least one additional therapeutic agent.

Typically, the additional therapeutic agent(s) present in the pharmaceutical composition implemented in the invention may be therapeutic agent(s) already used in the prevention and/or the treatment of a viral infection caused by SARS-CoV-2 and therapeutic agent(s) already used in the prevention and/or the treatment of symptoms or complications encountered in a viral infection caused by SARS-CoV-2. Advantageously, the additional therapeutic agent(s) present in the pharmaceutical composition implemented in the invention is/are selected in the group consisting of anti-viral agents, anti-inflammatory agents, analgesic agents, muscle-relaxant agents, anaesthetic agents, diuretic agents and antibiotic agents.

When the pharmaceutical composition implemented in the invention comprises at least one additional therapeutic agent which is at least one anti-viral agent, it is clear that this at least one additional anti-viral agent is different from a compound of formula (I) or a pharmaceutically acceptable salt or solvate thereof.

A first example of anti-viral agent usable in the invention is QVE-OPh or a pharmaceutically acceptable salt or solvate thereof, in particular, such as disclosed in the International application WO 2021/228846. What has already been disclosed for a pharmaceutically acceptable salt or solvate of QVDM-OPh applies *mutatis mutandis* to a pharmaceutically acceptable salt or solvate of QVE-OPh. In other words, the at least one additional therapeutic agent of the pharmaceutical composition implemented in the invention may be the N-(2(quinolyl)-valyl-glutamyl-(2,6-difluorophenoxy)methyl ketone or a pharmaceutically acceptable salt or solvate thereof.

As other anti-viral agents usable in the invention, one can cite viral RNA-dependent RNA polymerase modulators such as nucleotide analogues and fusion inhibitors. The latter prevent the fusion between the SARS-CoV-2 envelope and the cell membrane and then the entry of SARS-CoV-2 into the cell. Advantageously, fusion inhibitors is a serine/protease inhibitors, inhibitors of angiotensin-converting enzyme 2 (ACE2) or antimalarial/parasiticide drugs. As a consequence, a fusion inhibitor usable in the invention is selected in the group consisting of remdesivir, camostat mesilate, nafamostat mesilate, chloroquine phosphate, hydroxychloroquine, cepharanthine, selamectin, and mefloquine and its salts such as mefloquine hydrochloride.

As anti-inflammatory agents usable in the invention, one can cite monoclonal antibodies. The latter are preferably directed against inflammatory interleukins and their receptors such as IL-6 and its receptors. As a consequence, a monoclonal antibody usable in the invention is selected in the group consisting of tocilizumab, sarilumab, and siltuximab.

Alternatively or in addition, the additional therapeutic agent(s) present in the pharmaceutical composition implemented in the invention is/are selected in the group consisting of cisatracurium besylate, dexamethasone sodium phosphate, dexmedetomidine hydrochloride, fentanyl citrate, furosemide, hydromorphone hydrochloride, ketamine hydrochloride, lorazepam, midazolam hydrochloride, morphine sulfate, norepinephrine bitartrate, rocuronium bromide, vancomysin hydrochloride, and vecuronium bromide.

In a particular embodiment, the pharmaceutical composition implemented in the invention comprises at least two or at least three additional therapeutic agents selected in a same list or in different lists amongst the previously defined lists.

In a more particular embodiment, the pharmaceutical composition implemented in the invention comprises or consists of (i) the compound of formula (I) or a pharmaceutically acceptable salt or solvate thereof as previously defined, (ii) at least one pharmaceutically acceptable vehicle, (iii₁) at least one additional anti-viral agent such as previously defined and (iii₂) at least one anti-inflammatory agent such as previously defined.

A dosage of the pharmaceutical composition implemented in the invention needs to be a pharmaceutically effective amount. The "pharmaceutically effective amount" means an amount enough to prevent or treat diseases at a reasonable benefit/risk ratio applicable to medical treatment, and a level of effective dose may be variously selected by those skilled in the art according to factors such as, for example, a formulation method, a patient's condition including weight, gender and age, a degree of disease, a drug form, an administration route, an administration period if this administration implements single or fractionated doses, an excretion rate and reaction sensitivity. The effective amount may vary depending on a route of disposal, a use of excipients and possibility of being used with other therapeutic agent(s), as recognized by those skilled in the art.

The present invention also concerns a kit of parts comprising or consisting of:
a) a compound of formula (I) or a pharmaceutically acceptable salt or solvate thereof or a pharmaceutical composition comprising it as previously defined, and
b) at least one additional therapeutic agent, in particular such as previously defined or a pharmaceutical composition comprising at least one additional therapeutic agent,
for use in preventing and/or treating a viral infection caused by SARS-CoV-2.

What has already been disclosed for the pharmaceutical composition comprising a compound of formula (I) or a pharmaceutically acceptable salt or solvate thereof applies *mutatis mutandis* to the pharmaceutical composition comprising at least one additional therapeutic agent.

A kit of parts can also be defined as a combination of the elements a) and b) as above defined.

Such a kit of parts is of particular interest in the invention when the compound of formula (I) or a pharmaceutically acceptable salt or solvate thereof and the at least one additional therapeutic agent cannot be formulated in the same pharmaceutical composition and/or when the compound of formula (I) or a pharmaceutically acceptable salt or solvate thereof or the pharmaceutical composition comprising it and the at least one additional therapeutic agent or the pharmaceutical composition comprising it are to be administrated separately or sequentially.

Thus, the kit of parts as implemented in the invention can be used simultaneously, separately or sequentially and in particular for preventing and/or treating a viral infection caused by SARS-CoV-2.

As used herein, a simultaneous use means that the element a) as above defined is administrated as the same time as the element b) as above defined to a patient. The zone of administration at the level of the patient and thus the administration route can be identical or different.

As used herein, a separate or sequential use means that the elements a) and b) as above defined are administrated separately or sequentially provided that the time period during which the element a) exerts its pharmacological effects on the patient and the time period during which the element b) exerts its pharmacological effects on the patient at least partially intersect.

As used herein, a "patient", a "patient in need", a "subject" and a "subject in need" mean either a subject at risk of developing a viral infection caused by SARS-CoV-2 or a subject already contaminated by SARS-CoV-2. The subject may be a non-human animal or a human. The contamination can be confirmed by the detection of proteins of SARS-CoV-2 or of antibodies specific to SARS-CoV-2 in samples derived from the subject such as saliva, blood or nasopharyngeal sample. The subject contaminated by SARS-CoV-2 may be symptomatic, paucisymptomatic or asymptomatic. The subject in need may also be a subject suffering from mid- and long-term effects after recovering from the initial illness. These mid- and long-term effects are designated as "post COVID-19 condition" or "long COVID" and more especially as "Neuro-Long COVID".

In addition, the compound of formula (I) or a pharmaceutically acceptable salt or solvate thereof for use as previously defined, the pharmaceutical composition for use as previously defined and/or the kit of parts for use as previously defined is implemented for use in preventing, inhibiting and/or reducing viral replication and/or viral protein synthesis in a subject infected by SARS-CoV-2. In this embodiment, the subject may also be a non-human animal or a human. The prevention or inhibition of viral replication and/or of viral protein synthesis can be either partial or total.

The expression "viral replication" as used herein includes the totality of the steps of the replication cycle of the virus. In particular this expression includes the main steps of replication of the SARS-CoV-2, including entry of the virus into the cell, formation of the viral replication and transcription complex, viral genomic RNA replication, formation of the structural proteins by transcription and translation of the negative template and assembly of viral particles.

The ability of the compound of formula (I) or a pharmaceutically acceptable salt or solvate thereof, of a pharmaceutical composition or of a kit of parts as previously defined to prevent, inhibit and/or reduce viral replication and/or viral protein synthesis can be evaluated, for example, *in vitro.* The hereinafter example 1 proposes techniques for this evaluation including western blot analysis and qRT-PCR analysis.

Moreover, the compound of formula (I) or pharmaceutically acceptable salt or solvate thereof for use as previously defined, the pharmaceutical composition for use as previously defined and/or the kit of parts for use as previously defined is implemented for use in preventing complications of COVID-19 such as COVID-19 pneumonia, acute respiratory distress syndrome (ARDS), acute respiratory failure and liver or cardiac injury.

The compound of formula (I) or a pharmaceutically acceptable salt or solvate thereof such as previously defined, the pharmaceutical composition such as previously defined or the kit of parts such as previously defined may be used in a method for preventing and/or treating a viral infection caused by SARS-CoV-2 in a patient in need thereof, wherein said method comprises a step of administrating said compound, pharmaceutical composition or kit of parts to the patient in need thereof.

As already disclosed for the pharmaceutical composition, the compound of formula (I) or a pharmaceutically acceptable salt or solvate thereof and the elements a) and b) of the kit of parts can be administered by the systemic route; by the parenteral route, for example the intravenous, intra-arterial, intraperitoneal, intrathecal, intraventricular, intrasternal, intracranial, intramuscular or sub-cutaneous route; by the topical route; by the ocular route; by the oral route and by the mucosal route such as the buccal, nasal, intranasal, rectal and vaginal routes.

In addition, as already disclosed for the pharmaceutical composition, the compound of formula (I) or a pharmaceutically acceptable salt or solvate thereof and the elements a) and b) of the kit of parts are to be administrated in pharmaceutically effective amounts. The definition previously given for pharmaceutical composition applies *mutatis mutandis* to the compound of formula (I) or a pharmaceutically acceptable salt or solvate thereof and the elements a) and b) of the kit of parts.

In a particular embodiment, the compound of formula (I) or a pharmaceutically acceptable salt or solvate thereof, the pharmaceutical composition or the kit of parts may be used for preventing, inhibiting and/or reducing viral replication and/or viral protein synthesis, in a subject infected by SARS-CoV-2.

In another particular embodiment, the compound of formula (I) or a pharmaceutically acceptable salt or solvate thereof, the pharmaceutical composition or the kit of parts may be used for preventing complications of COVID-19 such as COVID-19 pneumonia, acute respiratory distress syndrome (ARDS), acute respiratory failure and liver or cardiac injury, in particular in a patient in need thereof and, more particularly, in a subject infected by SARS-CoV-2.

Other characteristics and advantages of the present invention will additionally be apparent to the one skilled in the art on reading the examples below, which are given as an illustration and not a limitation, with reference to the attached figures.

### BRIEF DESCRIPTION OF THE FIGURES

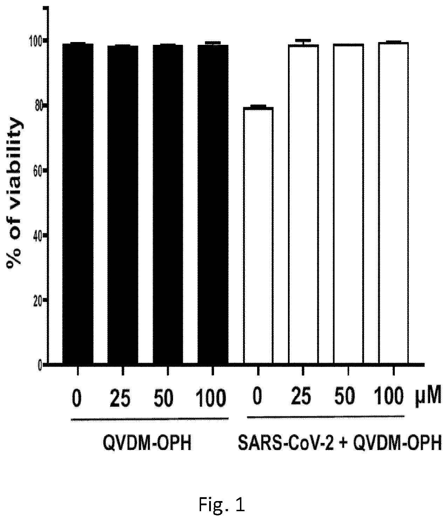

**Figure 1****: Toxicity test for QVDM-OPh peptide on Vero E6/TMPRS22 cells** Vero-E6/TMPRSS2 cells non infected or infected with the SARS-CoV-2 at MOI 0.05 and incubated with different concentrations of QVDM-OPh at the concentration of 25, 50, 100 µM for each day and were collected after 72 h. Cells were washed twice with PBS before viability fixable dye staining for 30 min at 4°C, then washed and fixed with 2% paraformaldehyde (PFA) and analyzed on a Fortessa Flux Cytometer. 30 000 events were recorded for each condition in triplicate. Analyses were done using a FlowJo Software and the percentages of viability were calculated according to the analysis report. Results represent mean +/- SD of 4 independent experiments with 3 independent points for each condition separately.

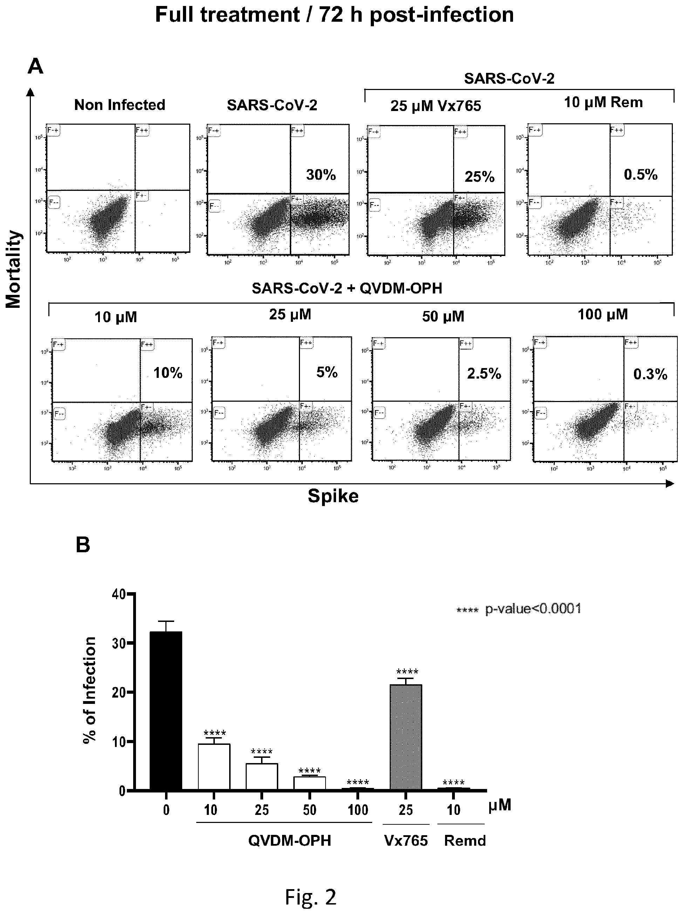

**Figure 2****: Effect of QVDM-OPh peptide on infection and mortality induce by SARS-CoV-2 for the full treatment condition. Flow cytometry analysis for the intracellular detection of the Sars-CoV-2 Spike (S) protein expression in infected cells with no treatment** or **treated with the different compounds.**
   **A)** Vero E6/TMPRSS2 cells non infected or infected with the SARS-CoV-2 at MOI 0.05 were incubated with different concentrations of QVDM-OPh (10, 25, 50, 100 µM), Vx765 (25 µM) or Remdesivir (10 µM) for 1 h, before the infection. Afterwards, the cells were cultured with drug-containing medium until the end of the experiment (Full treatment) without removing the virus from the culture. After 72 h post-infection, cells were collected and stained for mortality and infection rate analysis. The % of infection is represented in each plot of analysis.
   **B)** Results represent the % of infection detected by the Spike protein staining in the different infected and treated cells compared to the infected untreated group. Results represent mean +/- SD of 4 independent experiments with 3 independent points for each condition separately. Statistical significance was assessed applying *One-way ANOVA test followed by Dunnett's post-hoc test* using GraphPad Prism software (GraphPad Software Inc., USA). (****P < 0.0001).

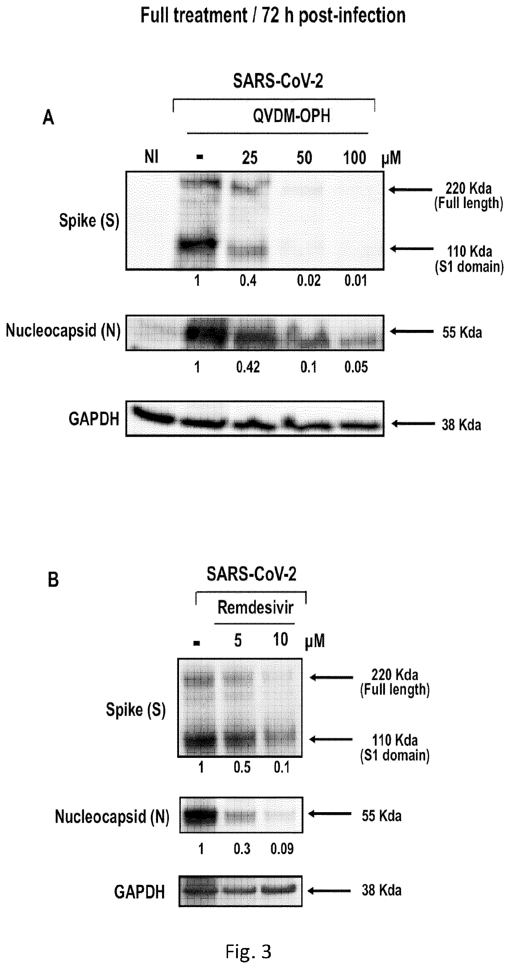

**Figure 3****: Effect of QVDM-OPh peptide on viral replication and viral protein expression *in vitro* for the full treatment condition. Western blot analysis for the intracellular detection of the Sars-CoV-2 Spike (S) and nucleocapsid (N) proteins in infected cells with no treatment or treated with the different compounds.**
   **A-B)** Vero E6/TMRPSS2 cells were pre-treated with QVDM-OPh peptide or Remdesivir at the indicated concentrations for 1 h, before the infection with the virus at MOI = 0.05. Afterwards, the cells were cultured with drug-containing medium until the end of the experiment (Full treatment). A well with non-infected cells was performed as a negative control of the infection. At 72 h post-infection, cells were lysed by RIPA buffer and western blot analysis was performed to detect the expression of the Spike protein (S), the full length and S1 domain, and the Nucleocapsid protein (N). GAPDH was used as loading control. Results represent mean ± SD, from 4 independent experiments with 3 independent points per condition.

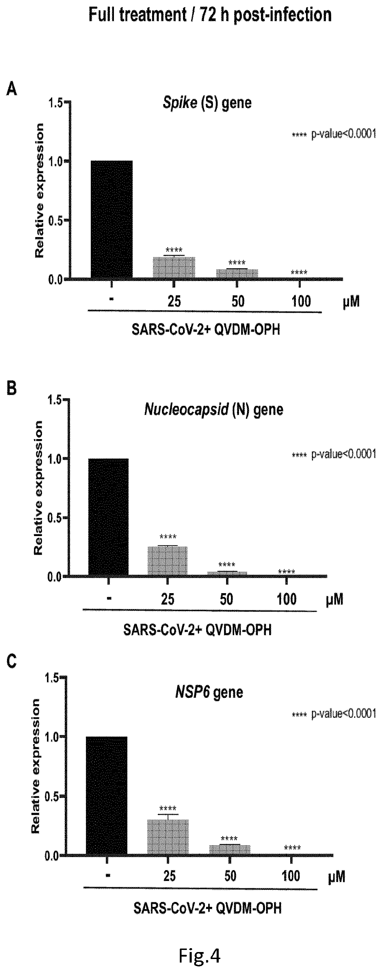

**Figure 4****: Effect of QVDM-OPh peptide on viral replication *in vitro* for the full treatment condition. Intracellular qRT-PCR analysis for the Sars-CoV-2 Spike (S), nucleocapsid (N) and NSP6 genes in infected cells with no treatment or treated with QVDM-OPH.**
   **A-B-C)** Vero E6/TMRPSS2 cells were pre-treated with QVDM-OPH peptide at the indicated concentrations for 1 h, before the infection with the virus at MOI = 0.05. Afterwards, the cells were cultured with drug-containing medium until the end of the experiment (Full treatment). A well with non-infected cells was performed as a negative control of the infection. At 72 h post-infection, cells were lysed in LBP buffer for RNA purification and RT-qPCR analysis was performed to detect the gene expression of the Spike protein (S), the Nucleocapsid protein (N) and NSP6. Relative mRNA quantities for each gene were normalized to GAPDH mRNA expression. Fold change was calculated using the ΔΔCt method. Results represent mean ± SD, from 4 independent experiments with 3 independent points per condition.
**Figure 5****: The antiviral activity of QVDM-OPh peptide against SARS-CoV-2 *in vitro* for the full treatment condition. Virus yield in the infected cell supernatants was quantified by qRT-PCR.**
   **A-B)** Vero E6/TMRPSS2 cells were pre-treated with QVD-OPh (QVD), QVDM-OPh (QVDM) or Remdesivir at the indicated concentrations for 1h, before the infection with the virus at MOI = 0.05. A well with non-infected cells was performed as a negative control of the infection. Afterwards, the cells were cultured with drug-containing medium until the end of the experiment (Full treatment). At 72 h post-infection, supernatants were collected, and viral RNA was extracted. Real-time PCR analysis was performed on supernatant using probes against either the SARS-CoV-2 N gene or NSP6 gene. Results represent mean + SEM (n=3). Comparisons of differences between means were explored using *One-way ANOVA test followed by Dunnett's post-hoc test. ***p<0.001 compared to the untreated group.*

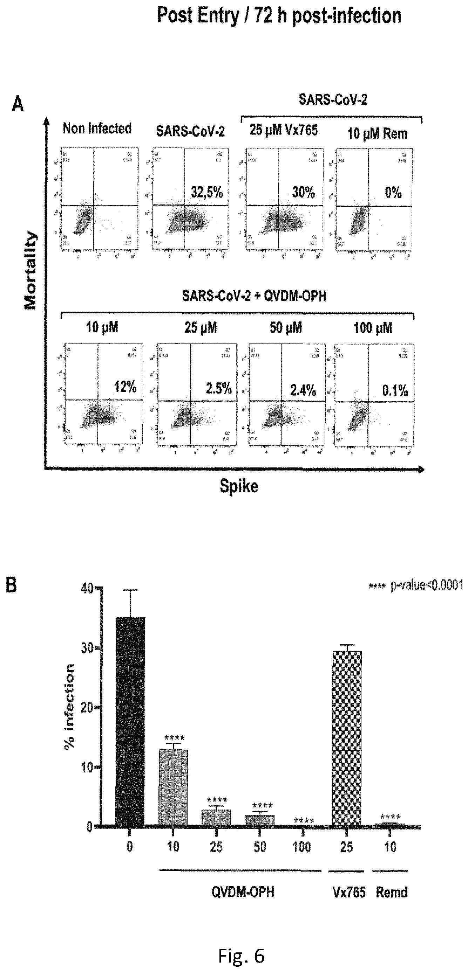

**Figure 6****: Effect of QVDM-OPh peptide on infection and mortality induce by SARS-CoV-2 for the post-entry treatment condition. Flow cytometry analysis for the detection of the expression of Sars-CoV-2 Spike (S) protein in infected cells with no treatment, or treated with the different compounds.**
   **A)** Vero E6/TMPRSS2 were infected with the virus at MOI 0.05 for 2 h and then the virus was removed from the medium. Then, the cells were incubated with different concentrations of QVDM-OPh (10, 25, 50 and 100 µM), Vx765 (25 µM) or Remdesivir (10 µM) for 72 h (Post-Entry). The drugs were added each day at the different concentration medium until the end of the experiment. After 72 h post-infection cells were collected and stained for mortality and infection rate analysis. The % of infection is represented in each plot of analysis.
   **B)** Results represent mean + SEM of the % of inhibition of the expression of the Spike protein staining as compared to the untreated control group (n=3).

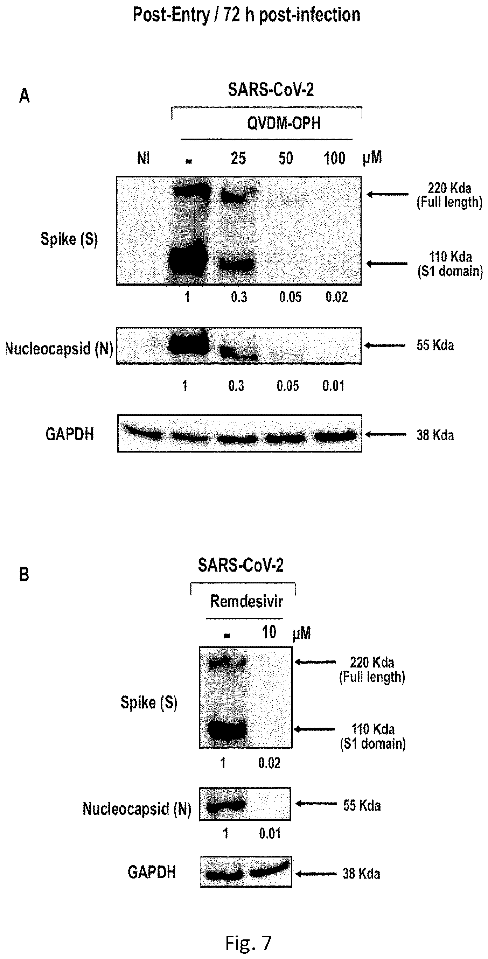

**Figure 7****: Effect of QVDM-OPh peptide on viral replication and viral protein expression *in vitro* for the post-entry treatment condition. Western blot analysis for the intracellular detection of the Sars-CoV-2 Spike (S) and nucleocapsid (N) proteins in infected cells with no treatment or treated with the different compounds.**
   **A-B)** Vero E6/TMPRSS2 cells were infected then treated with QVDM-OPh at the indicated concentrations or Remdesivir in the same conditions as described in **Figure 6****.** A well with non-infected cells was performed as a negative control of the infection. At 72 h post-infection, cells were lysed by RIPA buffer and western blot analysis was performed to detect the expression of the Spike protein (S), the full length and S1 domain, and the Nucleocapsid protein (N). GAPDH was used as loading control. Results represent mean ± SD, from 4 independent experiments with 3 independent points per condition.

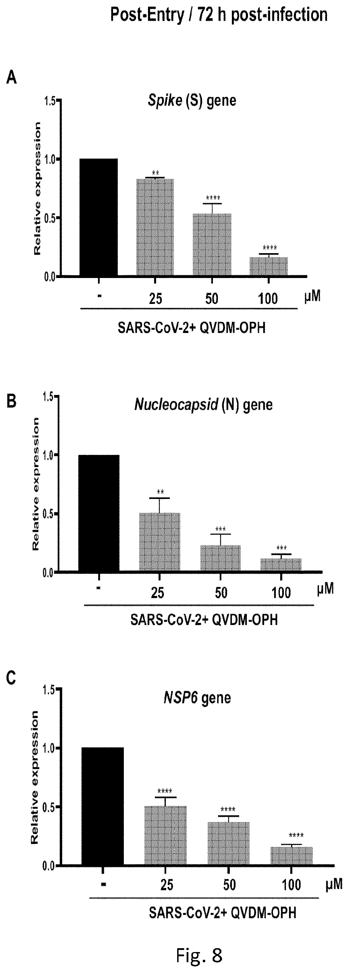

**Figure 8****: Effect of QVDM-OPh peptide on viral replication *in vitro* for the post-entry treatment condition. Intracellular qRT-PCR analysis for the Sars-CoV-2 Spike (S), nucleocapsid (N) and NSP6 genes in infected cells with no treatment or treated with QVDM-OPh.**
   **A-B-C)** Vero E6/TMPRSS2 cells were infected with the virus at MOI 0.05 for 2 h and then the virus was removed from the medium. Then, the cells were incubated with different concentrations of QVDM-OPh for 72 h (Post-Entry). The drugs were added each day at the different concentration medium until the end of the experiment. A well with non-infected cells was performed as a negative control of the infection. At 72 h post-infection, cells were lysed in LBP buffer for RNA purification and RT-qPCR analysis was performed to detect the gene expression of the Spike protein (S), the Nucleocapsid protein (N) and NSP6. Relative mRNA quantities for each gene were normalized to GAPDH mRNA expression. Fold change was calculated using the ΔΔCt method. Results represent mean ± SD, from 4 independent experiments with 3 independent points per condition.
**Figure 9****: The antiviral activity of QVDM-OPh peptide against SARS-CoV-2 *in vitro* for the post-entry treatment condition. Virus yield in the infected cell supernatants was quantified by qRT-PCR.**
   **A-B)** Vero E6/TMPRSS2 cells were infected with the virus at MOI 0.05 for 2 h and then the virus was removed from the medium. Then, the cells were incubated with different concentrations of QVD-OPh (QVD), QVDM-OPh (QVDM) or Remdesivir at the indicated concentrations (Post-Entry). The drugs were added each day at the different concentrations medium until the end of the experiment. A well with non-infected cells was performed as a negative control of the infection. At 72 h post-infection, supernatants were collected, and viral RNA was extracted. Real-time PCR analysis was performed on supernatant using probes against either the SARS-CoV-2 N gene or NSP6 gene. Results represent mean + SEM (n=3). Comparisons of differences between means were explored using *One-way ANOVA test followed by Dunnett's post-hoc test. ***p<0.001 compared to the untreated group.*
**Figure 10****: Effect of QVDM-OPh and QVE-OPh on SARS-CoV-2 in the COVID-19 golden Syrian hamster model**
   Golden Syrian hamsters were randomized on D-3, inoculated with SARS-CoV-2 at 10⁵ pfu TCID50 per animal. All groups have received SARS-CoV-2 by intranasal (IN) route on Day 0 (D0). QVDM-OPh and QVE-OPh peptides or the vehicle was administrated at D0 by intraperitoneal (IP) route under a total volume of 10 mL/kg of and per inoculation time point. The test items have been inoculated on D0 (t+1h) and Day 1 (D1). Day 2 (D2) was the last day of the study.
   **A)** Mean body weight change curves (vs D0) between groups of vehicle, QVE-OPH and QVDM-OPH treated animals at D0, D1 and D2 post-infection.
   **B)** Viral RNA expression levels (mean + SD) in lungs from golden Syrian hamsters inoculated with SARS-CoV-2 at 2 dpi, n = 5 per point. Results are expressed as 2^{-ΔCT}. Mann-Whitney test comparing infected animals to mock (A). *P < 0.05; **P < 0.01.
   C) Percentage of viral replication decrease in the lung obtained from golden Syrian hamsters inoculated with SARS-CoV-2 at 2 dpi, and treated with QVE-OPH or QVDM-OPH compared to vehicle.

### EXAMPLE 1

### MATERIALS & METHODS

### Cells, virus and drugs

African green monkey kidney Vero E6/TMPRSS2 cell line was obtained kindly from Dr. Andreola Marie-Aline, University of Bordeaux, and maintained in Eagle's medium (Dulbecco's modified Eagle's medium; Gibco Invitrogen supplemented with 10% heat-inactivated FBS, 1% PS (Penicillin 10,000 U/ml; Streptomycin 10,000 µg/ml) (Gibco Invitrogen) at 37°C in a humidified atmosphere of 5% CO2. The strain BetaCoV/France/IDF0372/2020 was supplied by the National Reference Centre for Respiratory Viruses hosted by Institut Pasteur (Paris, France) and headed by Pr. Sylvie van der Werf. The human sample from which strain BetaCoV /France/IDF0372/2020 was isolated, has been provided by Dr. X. Lescure and Pr. Y. Yazdanpanah from the Bichat Hospital, Paris, France. Moreover, the strain BetaCoV /France/IDF0372/2020 was supplied through the European Virus Archive goes Global (Evag) platform, a project that has received funding from the European Union's Horizon 2020 research and innovation program under grant agreement No. 653316. Viral stocks were produced on Vero-E6 cells infected at a multiplicity of infection of 1x 10-4 PFU. The virus was harvested 3 days after infection, clarified, and then aliquoted before storage at -80°C. Viral stocks were titrated on Vero-E6 cells by classical plaque assay using semisolid overlays (Avicel, RC581-NFDR080I, DuPont). All the infection experiments were performed in a biosafety level-3 (BLS-3) laboratory at the CRC (Cordelier Research Center). The pan-caspase inhibitor, Q-VD(OME)-OPH known as QVDM-OPH (Cat no. BIOV2787-5) and QVE-OPH were purchased from Avantor-VWR and Hello Bio. Remdesivir was purchased from COGER (Cat no. AG--CR1-3713-M005) and Vx765, caspase-1 inhibitor (Cat no. BIOV2781-5) from Avantor-VWR.

### Evaluation of antiviral activities, Toxicity and infection inhibition

To evaluate the toxicity of the QVDM-OPh on Vero E6 Cells and the antiviral efficacy, the percentage of mortality and the percentage of infected cells were measured by flow cytometry. Cells were cultured overnight in 24-well cell-culture petri-dish with a density of 75 × 10⁴ cells/well. The next day, cells were pretreated or not for 1 h with the different doses of the indicated QVDM-OPh, Vx765 or Remdesivir at different concentrations. Then, the virus was subsequently added at MOI 0.05 to allow infection for 2 h in 250 µl/well. Afterwards, complete media was added to cell culture to a final volume of 500 µl/well. Drugs were added each day at same concentration to cell culture. At 72 h post-infection, the cell supernatant was collected and frozen immediately at -80°C for viral extraction and qRT-PCR amplification. The cells were collected and a part thereof was used to flow cytometry analysis to measure the rate of infection using an intracellular staining against Spike protein (SARS-CoV-2 Spike Protein-Alexa 647, Cat no. 51-6490-82, eBioscience) and a Cytofix/cytoperm fixation permeabilization kit (Cat no. 554714, BD) according to the manufacturer's instructions. Toxicity was analysed using Viability 405/452 Fixable Dye (Cat no. 130-109-814, from Miltenyi Biotec) according to the manufacturer's instructions. Briefly, the cells were washed twice with PBS before viability fixable dye staining for 30 min at 4°C. Then, the cells were permeabilized by the Cytofix/cytoperm buffer for 20 min, and after two washes with the permawash buffer, the anti-spike-Alexa 647 was added to the cells for 30 min at 4°C. After the staining, the cells were fixed with 2% Paraformaldehyde (FPA) and then analyzed on a Fortessa Flow Cytometer. 30000 events were recorded for each condition in triplicate. Analyses were done using a FlowJo Software. The other part of the cells was lysed in RIPA lysis buffer (Invitrogen, Cat no. 10230544) containing protease (Roche) and phosphatase inhibitors (Invitrogen) for further quantification and immunoblotting analysis, or in LBP buffer for RNA purification and RT-qPCR analysis. Each condition was done in triplicate (n=3) in the same experiment and repeated for 4 independent experiments.

### Time-of-addition experiment

QVDM-OPh (10, 25, 50 and 100 µM), Vx765 (25 µM) and Remdesivir (5 and 10 µM) were used for the time-of-addition experiment. Vero E6 cells (75 × 10³ cells/well) were treated with QVDM-OPh, Vx765 and Remdesivir, at different stages of virus infection. For "Full-time" treatment, Vero E6 cells were pre-treated with the drugs for 1 h prior to virus infection, followed by incubation with virus for 2 h in the presence of the drugs until the end of the experiment. For "Post-entry" experiment, virus was added to the cells to allow infection for 2 h, and then virus-containing supernatant was replaced with drug-containing medium until the end of the experiment.

### RNA extraction and quantitative real-time RT-PCR (qRT-PCR) in Vero E6/TMPRSS2 cells

### Viral RNA extraction from supernatant

Two hundred microliter cell culture supernatant was used for viral RNA extraction using the MiniBEST Viral RNA/DNA Extraction Kit (Takara, Cat no. 9766) according to the manufacturer's instructions. RNA was eluted in 30 µL RNAse-free water.

### Intracellular RNA purification

After being washed with PBS, cells were lysed with LBP and stocked at -80°C. ARN purification was done using "Nucleospin RNA PLUS" kit according to the manufacturer's recommended procedures (Machery Nagel ref #740984.250). RNA was eluted in 30 µL RNAse-free water.

### Quantitative real-time RT-PCR (qRT-PCR)

Total RNA was converted to cDNA using PrimeScript RT Reagent Kit with gDNA Eraser (Takara, Cat no. RR047A), following the manufacturer's recommended procedures. Quantitative PCR was performed using TB Green Premix Ex Taq II (Takara Cat no. RR820A). Briefly, each reaction consisted of a total volume of 25 µL containing 1 µL of each primer [0.4 µM/µL], 2 µl of cDNA (5 ng/µL), 12.5 µl TB Green Premix Ex Taq II and 8.5 µL of RNAse-free water.

Real-time PCR was performed using Bio Rad CFX384 Real-Time system PCR Machine. The thermal cycling conditions used were as follows : initial denaturation : 95°C for 30 s, followed by 40 cycles of amplification at 96°C for 5 s, and 60°C for 30 s. The primers used for SARS-CoV-2 Nucleocapsid (N), Non-Structural Protein 6 (NSP6) and Spike (S) genes designed and described by Abdel-Sater et al, 2021 ("A Rapid and Low-Cost protocol for the detection of B.1.1.7 lineage of SARS-CoV-2 by using SYBR Green-Based RT-qPCR", medRxiv preprint doi: https://doi.org/10.1101/2021.01.27.21250048) were purchased from Eurofins. For extracellular viral mRNA relative quantification, SARS-CoV-2 cDNA (Ct^{~}20 for N and NSP6 genes) was used as a positive control. Calculated Ct values were converted to fold-reduction of treated samples compared to the positive control using the ΔCt method (fold changed in viral RNA=2^ΔCt).

For intracellular mRNA, threshold cycle (Ct) values were obtained for each gene using the instrument software and auto-Ct function. Relative mRNA quantities for each gene were normalized to GAPDH mRNA expression. Fold change was calculated using the ΔΔCt method. Specific SARS-CoV-2 (N, NSP6 and S) and human (GAPDH) primers sequences used in the experiments are as follows:
N-qF: CGTTTGGTGGACCCTCAGAT (SEQ ID NO: 1 in the sequence listing) ;
N-qR: CCCCACTGCGTTCTCCATT (SEQ ID NO: 2 in the sequence listing) ;
NSP6-qF: GGTTGATACTAGTTTGTCTGGTTTT (SEQ ID NO: 3 in the sequence listing) ;
NSP6-qR: AACGAGTGTCAAGACATTCATAAG (SEQ ID NO: 4 in the sequence listing) ;
S-qF: GGTTCCATGCTATACATGTCTC (SEQ ID NO: 5 in the sequence listing) ;
S-qR: GGTCTTCGAATCTAAAGTAGTACCA (SEQ ID NO: 6 in the sequence listing) ;
GAPDH-qF: AAGGTCGGAGTCAACGGATTT (SEQ ID NO: 7 in the sequence listing) ;
GAPDH-qR: TGAAGGGGTCATTGATGGCA (SEQ ID NO: 8 in the sequence listing).

### Western blot analysis

For Western blot analysis, 40 µg of proteins from each point were resolved on 4-12% Bis-Tris-SDS-NUPAGE gels (Invitrogen) and then transferred onto nitrocellulose membranes (Amersham Bioscience). After being blocked with 5% BSA in TBS buffer containing 0.05% Tween 20, the membranes were then probed with mouse anti-Spike (S1-NTD) (E7M5X) (Ozyme, Cat. No. 42172S), rabbit anti-Nucleocapsid (Fisher Scientific, Cat. No. MA536086). Equal protein loading was assessed by probing the membranes with rabbit anti-GAPDH (Protein Tech, Cat. No. 60004-1-AP). Membranes were treated with horseradish peroxidase-linked goat anti-mouse or anti-rabbit secondary antibodies (Amersham Biosciences). Immunoreactive proteins were detected and quantified by enhanced chemiluminescence (Pierces, USA) using a CCD camera (GBOX, SYNGENE Pxi-4, Ozyme).

### RESULTS

Treatments with different concentrations of QVDM-OPh peptide have shown no toxicity effect on Vero E6/TMPRSS2 cells whether they were infected or not with the virus at MOI 0.05 for 72 h post-infection (Figure 1).

The antiviral and mortality effects of different concentrations of QVDM-OPh have been evaluated by flux cytometry analysis using intracellular staining against SARS-CoV-2 spike proteins and the viability dye to measure the mortality. Remdesivir was used as a positive control during the study and Vx765 as a negative control. The effect of QVDM-OPh on SARS-CoV-2 replication has also been evaluated by western blot analysis of viral protein expression, for Spike and Nucleocapsid proteins. The results obtained by flux cytometry and western blot show a significant inhibition of the viral replication by QVDM-OPh at the dose of 10 µM (inhibition is approximately 50%) and at 25 µM (inhibition is around 70%). The inhibition is complete at the concentration of 50 µM (inhibition is around 99%). This effect has been seen with daily doses during 72 h post-infection with a continuous presence of the virus in the culture (Full treatment condition). This reduction is comparable to that obtained with Remdisivir (Figures 2 and 3).

Then, the inventor has looked for the mRNA expression of SARS-CoV-2 viral genes, and, more particularly, for the gene expression of spike (S), nucleocapsid (N) and non-structural protein 6 or accessory protein ORF6 (NSP6) within infected cells treated or not with QVDM-OPh. Indeed, QVDM-OPh treatment is able to reduce significantly the relative expression of the structure protein, the Spike, the Nucleocapsid and the NSP6 genes inside the cells (Figure 4).

Finally, the inventor has verified if QVDM-OPh had inhibited the secretion/production of the virus from the cell to the media. For that, she has analyzed the supernatants of infected cells treated or not with different inhibitors for full treatment condition after viral extraction and qRT-PCR for the Nucleocapsid (N) and NSP6 gene expression The SARS-CoV-2 is significantly reduce in the supernatant of Vero E6/TMPRSS2 infected and QVDM-OPh treated cells like Remdesivir (Figure 5).

All these results have been reconducted in Post-Entry treatment condition, where the cells have been infected for 2 h and then the viruses have been completely removed from the media that has been replaced by a fresh one. All the drugs have been added after the 2 h of infection at the different concentrations and have been added daily during 72h. Experiences conducted in post-entry conditions show the same effect described in all experiences in full treatment conditions (Figures 6, 7, 8 and 9).

The present inventor's findings demonstrate that QVDM-OPh peptide is highly effective in the control of SARS-CoV-2 infection *in vitro* by inhibiting the viral replication inside the cell and preventing viral production and new infections without any toxicity even at the concentration of 25 µM and 50 µM.

In addition, when comparison has been made between QVDM-OPh and QVD-OPh used at an identical concentration (Figures 5 and 9), QVDM-OPh is able to inhibit the secretion/production of the virus from the cell to the media more efficiently than QVD-OPh implemented at the same concentration.

### EXAMPLE 2

### MATERIALS & METHODS

### COVID-19 golden Syrian hamster model

### 1. Ethical statement

Animal housing and experimental procedures have been conducted according to the French and European Regulations and the National Research Council Guide for the Care and Use of Laboratory Animals. The animal facility is authorized by the French authorities (Agreement N° B 91962 106). All animal procedures (including surgery, anesthesia and euthanasia as applicable) used in the current study have been submitted to the Institutional Animal Care and Use Committee of Oncodesign (Oncomet) approved by French authorities (CNREEA agreement N° 91).

The animal BSL3 facility is authorized by the French authorities (Agreement N° D92-032-02). All animal procedures (including surgery, anesthesia and euthanasia as applicable) used in the current study have been submitted to the Institutional Animal Care and Use Committee of CEA approved by French authorities (CETEA DSV - n° 44).

### 2. Housing conditions

Animals have been maintained in specific-pathogen free health status according to the FELASA guidelines. Animals have been individually identified. Animals will be maintained in housing rooms under controlled environmental conditions i.e. temperature: 21°C ± 2°C, humidity: 55 ± 10%, photoperiod (12 h light/12 h dark), H14 filtered air and minimum of 12 air exchanges per hour with no recirculation.

Each cage has been labeled with a specific code. Animal enclosures provided sterile and adequate space with bedding material, food and water, environmental and social enrichment (group housing) as described below: A3 facility: IsoRat900N biocontainment system (Techniplast, France), Poplar bedding (Select fine, Safe, France), A04 SP-10 diet (Safe, France), Tap water and Environmental enrichment with tunnel and wood sticks.

### 3. SARS-CoV-2

- SARS-CoV-2 strain "Slovakia/SK-BMC5/2020", originally provided by the European Virus Archive global (EVAg) (GISAID EPI_ISL_417879, https://www.european-virus-archive.com/virus/sars-cov-2-strainslovakiask-bmc52020), produced and tittered by Oncodesign on Vero E6/TMPRSS2 cells, has been used for hamster infection. The strain belongs to the GH clade.
- Virus production has been performed in T175 flasks seeded with 50x10⁶ Vero E6/TMPRSS2 cells and in a 40 mL final volume. Cell counts and viability have been assessed by 0.25% trypan blue exclusion assay by ViCell apparatus. After 48 h of infection time frame (with 0.001-0.005 MOI of SARS-CoV-2 virus), cytopathogenic effects have been confirmed under microscope observation. Culture supernatant has been harvested, centrifuged (5 min at 5000g) and aliquoted (1 mL aliquots).
- Virus stock TCID50 titers have been determined on Vero E6/TMPRSS2 cells. About 2 h before testing, cells have been plated in 96-well plate at the density of 2x10⁴ cells per well in a volume of 200 µL of complete growth medium (DMEM 10% FCS). Cells have been infected with serial dilutions of virus stock (8-plicates; 1st dilution 1:100; 5-fold serial dilutions) for 1 h at 37°C. Fresh medium has been added for 72 h and a MTS/PMS assay has been then performed, according to provider protocol (Promega, reference #G5430). Plates have been read using an ELISA Plate reader and data recorded. Infectivity has been expressed as TCID50/mL/72h based on the Spearman-Karber formula.

### Experimental Design and Treatments

### 1. Animals

12 healthy Golden Syrian Hamsters (females), 6-8 weeks old upon receipt, were obtained from Janvier Labs.

### 2. Randomization

Animals have been weighed before being allocated into 2 homogeneous groups of 5 animals. Animals have been labeled on the tail.

### 3. Treatments

The treatment has been administered by intraperitoneal (IP) route under a total volume of 10 mL/kg and per inoculation time point. The test item has been inoculated on Day 0 (t+1h) and Day 1. Treatment with test substance has been performed following the schedules indicated below:
- Group A animals (vehicle) Inoculations have been performed using the intraperitoneal (IP) route (10 mL/kg volume) using sodium chloride 0.9% supplemented with 2.5% DMSO;
- Group B animals (Q-VDM-OPH) have received the test compound (10 mL/kg volume, 10 mg/kg dosing) twice by IP route on Day 0 (t+1h) and Day 1;
- Group C animals (Q-VE-OPH) have received the test compound (10 mL/kg volume, 10 mg/kg dosing) twice by IP route on Day 0 (t+1h) and Day 1.

### 4. SARS-CoV-2 challenge

The administration route of SARS-CoV-2 has been chosen by Oncodesign. The virus has been administered by intranasal route (IN) under a total volume of 70 µL (35 µL per nostril) on Isoflurane-anesthetized animals. An intranasal dose of 10⁵ pfu TCID50 per animal has been administered. All groups have received SARS-CoV-2 by IN route on Day 0.

### 5. Termination of animals on Day 2 post-infection (2dpi)

The animals (n=12) have been terminated on Day 2 post-infection (2dpi). Animals have been deeply anesthetized using a cocktail of Zoletil (30 mg/kg - 0.6 mL/kg) and Xylazine (10 mg/kg - 0.5 mL/kg) injected by IP route. Gentle cervical dislocation followed by thoracotomy before lung collection.

Superior right lobe has been put in RNAlater overnight at 4°C, then stored at -80°C until RNA extraction for quantification of viral load by qRT-PCR. Middle, post caval and inferior right lobes will be snap frozen in liquid nitrogen (one lobe per tube), then stored at -80°C until further use.

### Ex vivo analysis

### 1- Virus load determination in lungs by genomic RT-qPCR

Quantification of viral load by RT-qPCR has been done from lung using viral ORFlab gene. Extraction of viral RNA has been performed using the Macherey Nagel Viral RNA kit (ref. 740452.4). RNA has been frozen at -80°C until qRT-PCR. Complete qRT-PCR has been run using SuperScript^{™} III One-Step qRT-PCR System kit (commercial kit #1732-020, Life Technologies) with primers and qRT-PCR conditions targeting ORFlab gene. Amplification has been performed using a Bio-Rad CFX384^{™}and adjoining software. The primers and probe implemented for this quantification are:
ORF1ab_Fw: CCGCAAGGTTCTTCTTCGTAAG (SEQ ID NO: 9 in the sequence listing);
ORF1ab_Rv: TGCTATGTTTAGTGTTCCAGTTTTC (SEQ ID NO: 10 in the sequence listing);
ORF1ab_probe: Hex-AAGGATCAGTGCCAAGCTCGTCGCC-BHQ-1 (SEQ ID NO: 11 in the sequence listing) with Hex at 5'-terminal position representing Hexachlorofluorescein and
BHQ-1 at 3'-terminal position representing Black Hole Quencher-1.

### 2- Animal monitoring

### Clinical monitoring

Animal viability, behavior and body weight have been monitored daily after SARS-CoV-2 infection. A model specific clinical follow-up has been recorded daily after SARS-CoV-2 infection, using the listed parameters below:
- Body weight loss
- Piloerection (absence - slight - marked)
- Behavior (normal - less mobile - amorphous or isolated)
- Posture (normal - abnormal - hunched)
- Cough (presence or absence)
- Sneeze (presence or absence)

### Humane endpoints

Humane endpoints have been established as follows:
- Twenty percent body weight loss (compared to the first day of treatment or maximum weight) lasting for a maximum of two consecutive days,
- Signs of pain, suffering or distress: pain posture, pain face mask, abnormal behavior or vocalization,
- Poor body condition, emaciation, cachexia, dehydration,
- Bladder outflow obstruction or diarrhea over a 48h period,
- Prolonged absence of voluntary responses to external stimuli,
- Rapid labored breathing,
- Anemia, significant bleeding,
- Bloodstained or mucopurulent discharge from any orifice,
- Neurologic signs: circling, convulsion, hind limb paralysis,
- Sustained decrease in body temperature,
- Abdominal distension.

### Anesthesia and analgesic

Isoflurane gas anesthesia has been used for test product inoculations and blood sampling at the jugular vein (when applicable). Non-pharmacological care has been provided for all painful procedures. Additionally, pharmacological care that does not interfere with the study (topical treatment) may be provided at the recommendation of the attending veterinarian.

### Euthanasia

Euthanasia of animals has been performed under deep anesthesia using a cocktail of Zoletil (30 mg/kg - 0.6 mL/kg) and Xylazine (10 mg/kg - 0.5 mL/kg) injected by IP route. Gentle cervical dislocation followed by thoracotomy and maximal terminal blood sampling has been performed before tissue collection. If physical methods for euthanasia (cervical dislocation) have been necessary, they have been performed by highly skilled and trained technicians.

### Statistics analysis

Statistical analysis was performed using Prism software (GraphPad, version 8, San Diego, USA). For *in vitro* experiments, *One-way ANOVA test followed by Dunnett's post-*hoc *test* has been used. For *in vivo* experiments, quantitative data have been compared across animal groups using Mann-Whitney test. Data represents Mean ± SEM. Statistics have been calculated based on One-way ANOVA test. *p<0.05, **p<0.01, ***p<0.001.

### RESULTS

To complete the studies performed *in vitro* on the antiviral effect of QVDM-OPh on SARS-CoV-2, the inventor has realized an *in vivo* assay in the COVID19 golden Syrian hamster model to show the efficacy of the QVDM-OPh peptide to inhibit the viral replication in lungs and compare it with QVE-OPh

The clinical scores and the body weight change vs Day 0 show encouraging results in infected and QVDM-OPh-treated group and in infected and QVE-OPh-treated group comparatively to the infected and untreated group (Figure 10A). Despite that the assay has been stopped at dpi2, and the animals have been euthanized at this time point, the results are very encouraging and show significant reduction of the viral load and the viral replication in the lungs of infected and treated animals comparatively to untreated groups (respectively Figure 10B and Figure 10C). In addition, the decrease in viral load and viral replication is more important for animals treated by QVDM-OPh (70% of viral replication decrease) compared to the ones treated with QVE-QVE-OPh (52% of viral decrease).

All these *in vitro* and *in vivo* results confirm an anti-viral effect of QVDM-OPh on SARS-CoV-2 replication.

## Claims

1. Compound of formula (I): 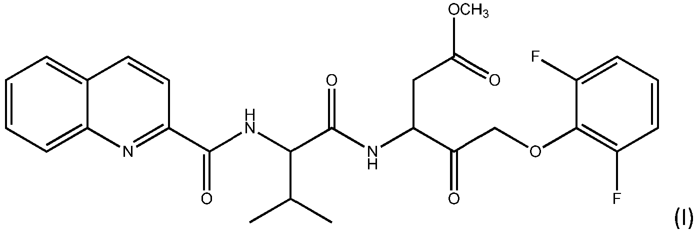 or a pharmaceutically acceptable salt or solvate thereof for use in preventing and/or treating a viral infection caused by SARS-CoV-2.

2. Pharmaceutical composition comprising, as active ingredient, the compound of formula (I) or a pharmaceutically acceptable salt or solvate as defined in claim 1, for use in preventing and/or treating a viral infection caused by SARS-CoV-2.

3. Pharmaceutical composition for use according to claim 2, wherein said pharmaceutical composition further comprises at least one pharmaceutically acceptable vehicle.

4. Pharmaceutical composition for use according to claim 2 or 3, wherein said pharmaceutical composition further comprises at least one additional therapeutic agent.

5. Kit of parts comprising or consisting of:
a) a compound of formula (I) or a salt or solvate thereof as defined in claim 1 or a pharmaceutical composition as defined in any one of claims 2 to 4, and
b) at least one additional therapeutic agent or a pharmaceutical composition comprising at least one additional therapeutic agent,
for use in preventing and/or treating a viral infection caused by SARS-CoV-2.

6. Pharmaceutical composition for use according to claim 4 or kit of parts for use according to claim 5, wherein said at least one additional therapeutic agent is selected in the group consisting of anti-viral agents, anti-inflammatory agents, analgesic agents, muscle-relaxant agents, anaesthetic agents, diuretic agents and antibiotic agents.

7. Pharmaceutical composition for use according to claim 4 or 6 or kit of parts for use according to claim 5 or 6, wherein said at least one additional therapeutic agent is the N-(2(quinolyl)-valyl-glutamyl-(2,6-difluorophenoxy)methyl ketone or a pharmaceutically acceptable salt or solvate thereof.

8. Pharmaceutical composition for use according to claim 6 or kit of parts for use according to claim 6, wherein said anti-viral agents are viral RNA-dependent RNA polymerase modulators or fusion inhibitors.

9. Pharmaceutical composition for use according to claim 6 or kit of parts for use according to claim 6, wherein said anti-inflammatory agents are monoclonal antibodies.

10. Compound of formula (I) or pharmaceutically acceptable salt or solvate thereof for use according to claim 1, pharmaceutical composition for use according to any one of claims 2-4 and 6-9 or kit of parts for use according to any one of claims 5-9,
wherein the compound of formula (I) or pharmaceutically acceptable salt or solvate thereof, the pharmaceutical composition and/or the kit of parts is implemented for use in preventing, inhibiting and/or reducing viral replication and/or viral protein synthesis in a subject infected by SARS-CoV-2.

11. Compound of formula (I) or pharmaceutically acceptable salt or solvate thereof for use according to claim 1, pharmaceutical composition for use according to any one of claims 2-4 and 6-9 or kit of parts for use according to any one of claims 5-9,
wherein the compound of formula (I) or pharmaceutically acceptable salt or solvate thereof, the pharmaceutical composition and/or the kit of parts is implemented for use in preventing complications of COVID-19 such as COVID-19 pneumonia, acute respiratory distress syndrome (ARDS), acute respiratory failure and liver or cardiac injury.

## Patentansprüche

1. Verbindung der Formel (I): 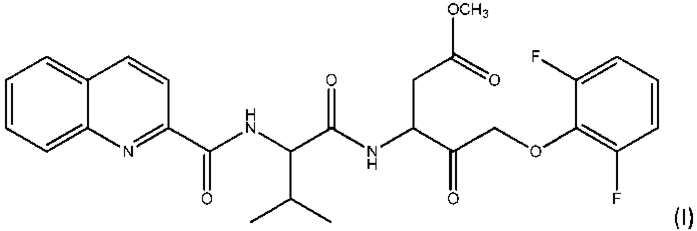 oder ein pharmazeutisch unbedenkliches Salz oder Solvat davon zur Verwendung bei der Prävention und/oder Behandlung einer Virusinfektion, die durch SARS-CoV-2 verursacht wird.

2. Pharmazeutische Zusammensetzung, die als Wirkstoff die Verbindung der Formel (I) oder ein pharmazeutisch unbedenkliches Salz oder Solvat nach Anspruch 1 umfasst, zur Verwendung bei der Prävention und/oder Behandlung einer Virusinfektion, die durch SARS-CoV-2 verursacht wird.

3. Pharmazeutische Zusammensetzung zur Verwendung nach Anspruch 2, wobei die pharmazeutische Zusammensetzung ferner mindestens ein pharmazeutisch unbedenkliches Vehikel umfasst.

4. Pharmazeutische Zusammensetzung zur Verwendung nach Anspruch 2 oder 3, wobei die pharmazeutische Zusammensetzung ferner mindestens ein zusätzliches therapeutisches Mittel umfasst.

5. Teilesatz, der Folgendes umfasst oder daraus besteht:
a) eine Verbindung der Formel (I) oder ein Salz oder Solvat davon nach Anspruch 1 oder eine pharmazeutische Zusammensetzung nach einem der Ansprüche 2 bis 4 und
b) mindestens ein zusätzliches therapeutisches Mittel oder eine pharmazeutische Zusammensetzung, die mindestens ein zusätzliches therapeutisches Mittel umfasst,
zur Verwendung bei der Prävention und/oder Behandlung einer Virusinfektion, die durch SARS-CoV-2 verursacht wird.

6. Pharmazeutische Zusammensetzung zur Verwendung nach Anspruch 4 oder Teilesatz zur Verwendung nach Anspruch 5, wobei das mindestens eine zusätzliche therapeutische Mittel ausgewählt ist aus der Gruppe, bestehend aus antiviralen Mitteln, entzündungshemmenden Mitteln, Analgetika, muskelrelaxierenden Mitteln, Anästhetika, Diuretika und Antibiotika.

7. Pharmazeutische Zusammensetzung zur Verwendung nach Anspruch 4 oder 6 oder Teilesatz zur Verwendung nach Anspruch 5 oder 6, wobei das mindestens eine zusätzliche therapeutische Mittel das N-(2-(Chinolyl)valylglutamyl-(2,6-difluorphenoxy))methylketon oder ein pharmazeutisch unbedenkliches Salz oder Solvat davon ist.

8. Pharmazeutische Zusammensetzung zur Verwendung nach Anspruch 6 oder Teilesatz zur Verwendung nach Anspruch 6, wobei die antiviralen Mittel virale RNA-abhängige RNA-Polymerase-Modulatoren oder Fusionsinhibitoren sind.

9. Pharmazeutische Zusammensetzung zur Verwendung nach Anspruch 6 oder Teilesatz zur Verwendung nach Anspruch 6, wobei die entzündungshemmenden Mittel monoklonale Antikörper sind.

10. Verbindung der Formel (I) oder pharmazeutisch unbedenkliches Salz oder Solvat davon zur Verwendung nach Anspruch 1, pharmazeutische Zusammensetzung zur Verwendung nach einem der Ansprüche 2 bis 4 und 6 bis 9 oder Teilesatz zur Verwendung nach einem der Ansprüche 5 bis 9,
wobei die Verbindung der Formel (I) oder das pharmazeutisch unbedenkliche Salz oder Solvat davon, die pharmazeutische Zusammensetzung und/oder der Teilesatz zur Verwendung bei der Prävention, Inhibierung und/oder Verringerung der viralen Replikation und/oder viralen Proteinsynthese in einem mit SARS-CoV-2 infizierten Individuum implementiert wird.

11. Verbindung der Formel (I) oder pharmazeutisch unbedenkliches Salz oder Solvat davon zur Verwendung nach Anspruch 1, pharmazeutische Zusammensetzung zur Verwendung nach einem der Ansprüche 2 bis 4 und 6 bis 9 oder Teilesatz zur Verwendung nach einem der Ansprüche 5 bis 9,
wobei die Verbindung der Formel (I) oder das pharmazeutisch unbedenkliche Salz oder Solvat davon, die pharmazeutische Zusammensetzung und/oder der Teilesatz zur Verwendung bei der Prävention von Komplikationen von COVID-19, wie COVID-19-Pneumonie, akutem Atemnotsyndrom (ARDS), akutem Atemversagen und Leber- oder Herzverletzung, implementiert wird.

## Revendications

1. Composé de formule (I) : 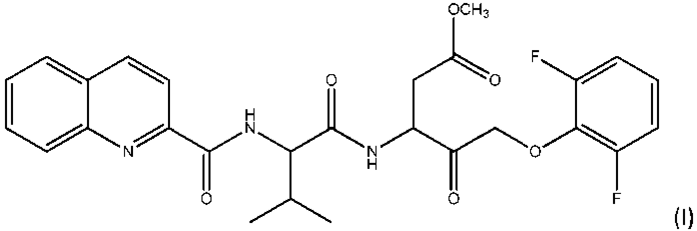 ou sel ou solvate pharmaceutiquement acceptable de celui-ci pour une utilisation dans la prévention et/ou le traitement d'une infection virale causée par le SARS-CoV-2.

2. Composition pharmaceutique comprenant, en tant qu'ingrédient actif, le composé de formule (I) ou un sel ou solvate pharmaceutiquement acceptable tel que défini dans la revendication 1, pour une utilisation dans la prévention et/ou le traitement d'une infection virale causée par le SARS-CoV-2.

3. Composition pharmaceutique destinée à être utilisée selon la revendication 2, dans laquelle ladite composition pharmaceutique comprend en outre au moins un véhicule pharmaceutiquement acceptable.

4. Composition pharmaceutique destinée à être utilisée selon la revendication 2 ou 3, dans laquelle ladite composition pharmaceutique comprend en outre au moins un agent thérapeutique supplémentaire.

5. Kit de composants comprenant ou constitué de :
a) un composé de formule (I) ou un sel ou solvate de celui-ci tel que défini dans la revendication 1 ou une composition pharmaceutique telle que définie dans l'une quelconque des revendications 2 à 4, et
b) au moins un agent thérapeutique supplémentaire ou une composition pharmaceutique comprenant au moins un agent thérapeutique supplémentaire,
pour une utilisation dans la prévention et/ou le traitement d'une infection virale causée par le SARS-CoV-2.

6. Composition pharmaceutique pour utilisation selon la revendication 4 ou kit de composants pour utilisation selon la revendication 5, où ledit au moins un agent thérapeutique supplémentaire est choisi dans le groupe constitué par des agents anti-viraux, des agents anti-inflammatoires, des agents analgésiques, des agents myorelaxants, des agents anesthésiques, des agents diurétiques et des agents antibiotiques.

7. Composition pharmaceutique destinée à être utilisée selon la revendication 4 ou 6 ou kit de composants destiné à être utilisé selon la revendication 5 ou 6, où ledit au moins un agent thérapeutique supplémentaire est la N-(2(quinolyl)-valyl-glutamyl-(2,6-difluorophénoxy))méthylcétone ou un sel ou solvate pharmaceutiquement acceptable de celle-ci.

8. Composition pharmaceutique pour utilisation selon la revendication 6 ou kit de composants pour utilisation selon la revendication 6, où lesdits agents antiviraux sont des modulateurs d'ARN polymérase ARN-dépendante virale ou des inhibiteurs de fusion.

9. Composition pharmaceutique pour utilisation selon la revendication 6 ou kit de composants pour utilisation selon la revendication 6, où lesdits agents anti-inflammatoires sont des anticorps monoclonaux.

10. Composé de formule (I) ou sel ou solvate pharmaceutiquement acceptable de celui-ci pour utilisation selon la revendication 1, composition pharmaceutique pour utilisation selon l'une quelconque des revendications 2 à 4 et 6 à 9 ou kit de composants pour utilisation selon l'une quelconque des revendications 5 à 9,
où le composé de formule (I) ou un sel ou solvate pharmaceutiquement acceptable de celui-ci, la composition pharmaceutique et/ou le kit de composants est mis(e) en œuvre pour une utilisation dans la prévention, l'inhibition et/ou la réduction de la réplication virale et/ou la synthèse de protéines virales chez un sujet infecté par le SARS-CoV-2.

11. Composé de formule (I) ou sel ou solvate pharmaceutiquement acceptable de celui-ci pour utilisation selon la revendication 1, composition pharmaceutique pour utilisation selon l'une quelconque des revendications 2 à 4 et 6 à 9 ou kit de composants pour utilisation selon l'une quelconque des revendications 5 à 9,
où le composé de formule (I) ou un sel ou solvate pharmaceutiquement acceptable de celui-ci, la composition pharmaceutique et/ou le kit de composants est mis(e) en œuvre pour une utilisation dans la prévention de complications du COVID-19 telles que la pneumonie COVID-19, le syndrome de détresse respiratoire aiguë (ARDS), l'insuffisance respiratoire aiguë et les lésions hépatiques ou cardiaques.
